(19) Europäisches Patentamt
European Patent Office
Office européen des brevets

(11) Veröffentlichungsnummer: **0 021 115**
**A1**

(12) # EUROPÄISCHE PATENTANMELDUNG

(21) Anmeldenummer: **80103023.0**

(22) Anmeldetag: **30.05.80**

(51) Int. Cl.³: **C 07 C 121/46**, C 07 C 121/16, C 07 C 121/30

(30) Priorität: **12.06.79 DE 2923778**

(43) Veröffentlichungstag der Anmeldung: **07.01.81**
**Patentblatt 81/1**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Hoffmann, Hellmut, Prof. Dr., Tersteegenweg 17, D-5600 Wuppertal 1 (DE)**
Erfinder: **Maurer, Fritz, Dr., Roeberstrasse 8, D-5600 Wuppertal 1 (DE)**
Erfinder: **Priesnitz, Uwe, Dr., Heinrich-Heine-Strasse 42, D-4750 Unna-Massen (DE)**
Erfinder: **Riebel, Hans-Jochem, Dr., In der Beek 92, D-5600 Wuppertal 1 (DE)**

(54) **Verfahren zur Herstellung von 3,3-Dimethyl-cyclopropan-1,1,2-tricarbonsäurederivaten sowie Alpha-Halogen-nitrile als neue Zwischenprodukte hierfür und Verfahren zu deren Herstellung.**

(57) Gegenstand der vorliegenden Erfindung sind ein Verfahren zur Herstellung von 3,3-Dimethyl-cyclopropan-1,1,2-tricarbonsäure-derivaten der Formel I

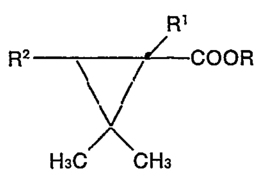

(I)

in welcher
R, $R^1$ und $R^2$ die in der Beschreibung angegebene Bedeutung haben, das dadurch gekennzeichnet ist,
daß man α-Halogencarbonsäure-derivate der Formel II

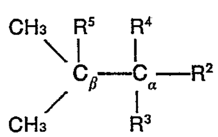

(II)

mit Malonsäurederivaten gegebenenfalls in Gegenwart einer Base und gegebenenfalls unter Verwendung eines Verdünnungsmittels bei Temperaturen zwischen 0 und 100° C umsetzt sowie neue Zwischenprodukte und Verfahren zu ihrer Herstellung.

EP 0 021 115 A1

BAYER AKTIENGESELLSCHAFT
Zentralbereich
Patente, Marken und Lizenzen

5090 Leverkusen, Bayerwerk

Rt/kl

Typ IV ZP

Verfahren zur Herstellung von 3,3-Dimethyl-cyclopropan-1,1,2-tricarbonsäurederivaten sowie α-Halogen-nitrile als neue Zwischenprodukte hierfür und Verfahren zu deren Herstellung

Die Erfindung betrifft ein neues Verfahren zur Herstellung von bekannten 3,3-Dimethyl-cyclopropan-1,1,2-tricarbonsäurederivaten, sowie α-Halogen-nitrile als neue Zwischenprodukte hierfür und Verfahren zu deren Herstellung.

Es ist bekannt, daß man 3,3-Dimethyl-cyclopropan-1,1,2-tricarbonsäure-triäthylester erhält, wenn man α,ß-Dibrom-ß,ß-dimethyl-propionsäure-äthylester mit Malonsäure-diäthylester in Gegenwart von Natriumäthylat umsetzt (vgl. J. Indian Chem. Soc. 14 (1937), 449-451).

Bei dieser Reaktion erhält man jedoch im allgemeinen auf einen Teil 3,3-Dimethyl-cyclopropan-1,1,2-tricarbonsäure-triäthylester etwa zwei Teile 3-Methyl-but-2-en-1,1,2-tricarbonsäure-triäthylester und etwa drei Teile 3-Methyl-but-3-en-1,1,2-tricarbonsäure-triäthylester; das gewünschte Produkt fällt also nur als Nebenprodukt an.

Weiter ist bekannt, daß man 3,3-Dimethyl-cyclopropan-1,1,2-tricarbonsäure-derivate, wie z.B. 3,3-Dimethyl-cyclopropan-1,1,2-tricarbonsäure-triäthylester oder 3,3-Dimethyl-cyano-cyclopropan-1,2-dicarbonsäure-diäthylester erhält, wenn man Isopropyliden-malonsäure-diäthylester bzw. Isopropyliden-cyanessigsäure-äthylester mit Dimethylsulfuranyliden-essigsäureäthylester in aprotischen Lösungsmitteln umsetzt (vgl. J. Org. Chem. 32 (1967), 3351-3355).
Wegen der Feuchtigkeitsempfindlichkeit von Dimethylsulfuranyliden-essigsäureäthylester müssen jedoch die Reaktionen zur Herstellung und Umsetzung dieser Verbindung in sorgfältig getrockneten Medien durchgeführt werden. Dimethylsulfuranyliden-essigsäureäthylester muß zudem bei tiefer Temperatur gelagert werden, da es sich bei Normaltemperatur allmählich zersetzt.

Ferner ist bekannt, daß man 3,3-Dimethyl-2-cyano-cyclopropan-1,1-dicarbonsäure-dimethylester oder -diäthylester erhält, wenn man (2-Brom-2-methyl-propyliden)- malonsäure-dimethylester oder -diäthylester mit Natrium- oder Kaliumcyanid umsetzt (vgl. Bull. Soc. Chim. Belg. 86 (1977), 55-63; ibid. 87 (1978), 721-732).
Die in der Literatur angegebene Synthese der als Ausgangsprodukte zu verwendenden (2-Brom-2-methyl- propyliden)-



BAD ORIGINAL

malonsäureester durch Umsetzung von (2-Methyl-propyliden)-malonsäureestern mit N-Brom-succinimid ist jedoch für die Herstellung im großtechnischen Maßstab wenig geeignet.

Gegenstand der vorliegenden Erfindung sind

(1) ein Verfahren zur Herstellung von 3,3-Dimethyl-cyclopropan-1,1,2-tricarbonsäure-derivaten der Formel I

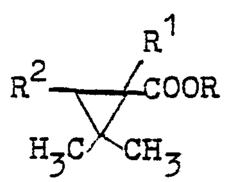
(I)

in welcher

R für Alkyl steht und

$R^1$ und $R^2$ für Alkoxycarbonyl oder Cyano stehen mit der Maßgabe, daß wenigstens einer dieser Reste für Cyano steht,

dadurch gekennzeichnet, daß man α-Halogencarbonsäure-derivate der Formel II

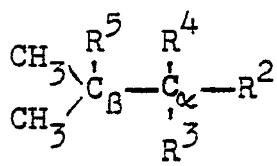
(II)

in welcher

$R^2$ für Alkoxycarbonyl oder Cyano steht,

$R^3$ für Brom oder Chlor steht,

$R^4$ für Wasserstoff steht oder zusammen mit $R^5$ für eine zusätzliche Bindung zwischen $C_\alpha$ und $C_\beta$ steht, und

$R^5$ für Brom oder Chlor steht oder zusammen mit $R^4$ für eine zusätzliche Bindung zwischen $C_\alpha$ und $C_\beta$ steht,

mit Malonsäurederivaten der Formel III

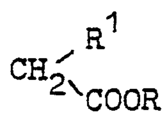

(III)

in welcher
R und $R^1$ die oben angegebenen Bedeutungen haben,

gegebenenfalls in Gegenwart einer Base und gegebenenfalls unter Verwendung eines Verdünnungsmittels bei Temperaturen zwischen 0 und 100°C umsetzt;

(2) neue $\alpha$-Halogen-nitrile der Formel IIa

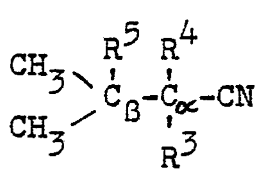

(IIa)

in welcher

$R^3$ für Brom oder Chlor steht,

$R^4$ für Wasserstoff steht oder zusammen mit $R^5$ für eine zusätzliche Bindung zwischen $C_\alpha$ und $C_\beta$ steht, und

$R^5$ für Brom oder Chlor steht, oder zusammen mit $R^4$ für eine zusätzliche Bindung zwischen $C_\alpha$ und $C_\beta$ steht;

(3) ein Verfahren zur Herstellung von α-Halogen-nitrilen der Formel IIa (oben), dadurch gekennzeichnet, daß man β,β-Dimethyl-acrylsäurenitril der Formel IV

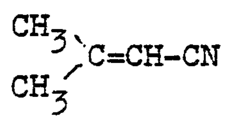 (IV)

mit Brom oder Chlor, gegebenenfalls in Gegenwart eines Verdünnungsmittels, bei Temperaturen zwischen -20 und +50°C umsetzt und gegebenenfalls die Halogenierungsprodukte der Formel IIa ($R^4$=H, $R^5$=Cl,Br) zur Eliminierung von Halogenwasserstoff bei Temperaturen zwischen 0 und 100°C mit Basen behandelt.

Überraschenderweise können nach den erfindungsgemäßen Verfahren (1) 3,3-Dimethyl-cyclopropan-1,1,2-tricarbonsäure-derivate der Formel (I) wesentlich einfacher und kostengünstiger als nach bekannten Methoden in guten Ausbeuten und in hoher Reinheit hergestellt werden.

Das neue Verfahren ist daher sehr gut zur industriellen Herstellung von 3,3-Dimethyl-cyclopropan-1,1,2-tricarbonsäure-derivaten geeignet.

Verwendet man als Ausgangsverbindungen beispielsweise α,β-Dichlor-iso-valeriansäure-methylester und Cyanessigsäure-äthylester, so kann der Reaktionsablauf beim erfindungsgemäßen Verfahren (1) durch folgendes Formelschema skizziert werden:

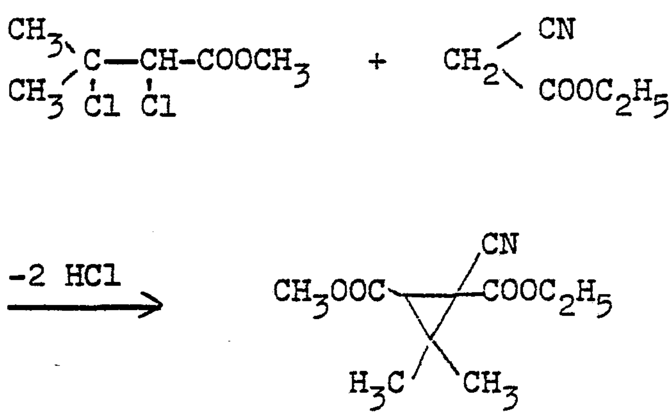


Die bei Verfahren (1) als Ausgangsverbindungen zu verwendenden α-Halogen-carbonsäure-derivate sind durch Formel (II) definiert. Vorzugsweise stehen darin

$R^2$ für $C_1$-$C_4$-Alkoxy-carbonyl oder Cyano,

$R^3$ für Brom oder Chlor

$R^4$ für Wasserstoff oder zusammen mit $R^5$ für eine zusätzliche Bindung zwischen $C_\alpha$ und $C_\beta$, und

$R^5$ für Brom oder Chlor, oder zusammen mit $R^4$ für eine zusätzliche Bindung zwischen $C_\alpha$ und $C_\beta$ .

Als Beispiele seien genannt:

α,ß-Dibrom-iso-valeriansäure-methylester, -äthylester, -n-propylester, -iso-propylester, -n-butylester, -iso-butylester, -sek.-butylester und -tert.-butylester,
α,ß-Dichlor-iso-valeriansäure-methylester, -äthylester, -n-propylester, -iso-propylester, -n-butylester, -iso-butylester, -sek.-butylester und tert.-butylester,
α-Brom-ß,ß-dimethyl-acrylsäure-methylester, -äthylester, -n-propylester, -iso-propylester, -n-butylester, -iso-butylester, -sek.-butylester und -tert.-butylester,
α-Chlor-ß,ß-dimethyl-acrylsäure-methylester, -äthylester, -n-propylester, -iso-propylester, n-butylester, -iso-butylester, sek.-butylester und tert.-butylester,
α,ß-Dibrom-isovaleriansäure-nitril,
α,ß-Dichlor-isovaleriansäure-nitril,
α-Brom-ß,ß-dimethyl-acrylsäure-nitril und
α-Chlor-ß,ß-dimethyl-acrylsäure-nitril.

Verbindungen der Formel (II), in welcher $R^2$ für Alkoxycarbonyl steht, sind bekannt (vgl. J. Chem. Soc. (London) 1944, 371-373; ibid. 1947, 750-752; ibid. 1949, 3089-3098; J. Am. Chem. Soc. 81 (1959), 2579-2588).

Man erhält Verbindungen der Formel (II), insbesondere die neuen Verbindungen der Formel (IIa), nach dem oben unter (3) beschriebenen Verfahren durch Umsetzung von ß,ß-Dimethyl-acrylsäure-derivaten, insbesondere von ß,ß-Dimethyl-acrylsäurenitril der Formel (IV), mit Brom

oder Chlor, gegebenenfalls in Gegenwart eines Verdünnungsmittels wie z.B. Tetrachlorkohlenstoff, bei Temperaturen zwischen -20 und +50°C, vorzugsweise zwischen 0 und 40°C, und gegebenenfalls anschließende Umsetzung des Halogenierungsproduktes mit einer Base wie z.B. Kaliumcarbonat, gegebenenfalls unter Verwendung eines Verdünnungsmittels wie z.B. Dimethylformamid, bei Temperaturen zwischen 0 und 100°C, vorzugsweise zwischen 10 und 80°C.

Die Aufarbeitung kann nach üblichen Methoden durchgeführt werden, beispielsweise durch Verdünnen mit Äther, Waschen mit Wasser, Trocknen, Filtrierung und gegebenenfalls Vakuumdestillation.

Das bei dem unter (3) beschriebenen Verfahren zur Herstellung neuer α-Halogen-nitrile der Formel (IIa) als Ausgangsverbindung einzusetzende ß,ß-Dimethyl-acrylsäurenitril (IV) ist bereits bekannt (vgl. US-PS 2 500 403).

Die weiter bei Verfahren (1) als Ausgangsstoffe zu verwendenden Malonsäure-derivate sind durch Formel (III) definiert. Vorzugsweise stehen darin:

R für $C_1$-$C_4$-Alkyl und

$R^1$ für $C_1$-$C_4$-Alkoxy-carbonyl oder Cyano.

Als Beispiele seien genannt:

Malonsäure-dimethylester, -diäthylester und -dipropylester sowie Cyanessigsäure-methylester, -äthylester, -n-propylester, -iso-propylester, -n-butylester, -iso-butylester, -sek.-butylester und -tert.-butylester.

Die Malonsäurederivate der Formel (III) sind bekannte Verbindungen.

Das erfindungsgemäße Verfahren (1) wird vorzugsweise unter Verwendung von Verdünnungsmitteln durchgeführt. Als solche kommen insbesondere polare organische Lösusungsmittel in Betracht. Hierzu gehören Carbonsäureamide, wie z.B. Dimethylformamid und N-Methylpyrolidon, Sulfoxide und Sulfone, wie z.B. Dimethylsulfoxid und Tetramethylensulfon, Phosphorsäureamide, wie z.B. Hexamethylphosphorsäuretriamid, Äther, wie z.B. Glycoldimethyläther, Diglycoldimethyläther, Tetrahydrofuran und Dioxan, Nitrile, wie z.B. Acetonitril und Propionitril, sowie Alkohole, wie z.B. Methanol, Äthanol, n-und iso-Propanol, n-, iso-, sek.- und tert.-Butanol.

Als Basen können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin, Pyridin und Diazabicyclononan.

Die Reaktionstemperatur kann innerhalb eines größeren Bereiches variiert werden. Im allgemeinen arbeitet man zwischen 0 und 100°C, vorzugsweise bei 10 bis 80°C.

Das erfindungsgemäße Verfahren wird im allgemeinen bei Normaldruck durchgeführt. Auf 1 Mol α-Halogen-carbonsäure-derivat der Formel (II) setzt man im allgemeinen 1 bis 3 Mol, vorzugsweise 1 bis 2 Mol Malonsäure-derivat der Formel (III) ein. In einer bevorzugten Ausführungsform des erfindungsgemäßen Verfahrens (1) wird das Malonsäurederivat (III) mit etwa der äquimolaren Menge einer der oben genannten Basen in einem der oben genannten Verdünnungsmittel vorgelegt und dieses Gemisch wird unter Rühren mit einem α-Halogen-carbonsäurederivat (II) tropfenweise versetzt. Das komplette Reaktionsgemisch wird mehrere Stunden bei Temperaturen zwischen 40 und 80°C gerührt. Die Aufarbeitung erfolgt auf übliche Weise: nach Abziehen des Lösungsmittels im Vakuum wird der Rückstand mit Wasser und mit einem mit Wasser nicht mischbaren Lösungsmittel, wie z.B. Äther, versetzt und geschüttelt. Die organische Phase wird abgetrennt, getrocknet, filtriert und eingeengt. Das zurückbleibende Rohprodukt kann durch Vakuumdestillation gereinigt werden. Zur Charakterisierung dient der Siedepunkt.

Die nach dem erfindungsgemäßen Verfahren (1) herzustellenden 3,3-Dimethyl-cyclopropan-1,1,2-tricarbonsäurederivate können durch partielle Hydrolyse, Decarboxylierung und erneute Hydrolyse gemäß folgendem Formelschema in 3,3-Dimethyl-cyclopropan-1,2-dicarbonsäure (Caronsäure) umgewandelt werden ($R^1$, $R^2$ : CN, COOAlkyl):

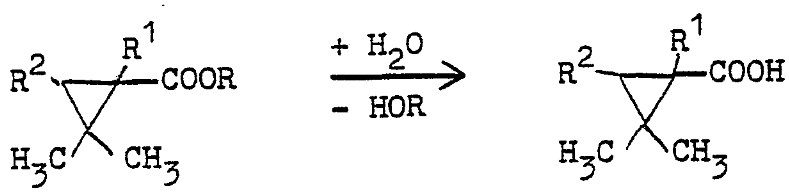


(I) (V)

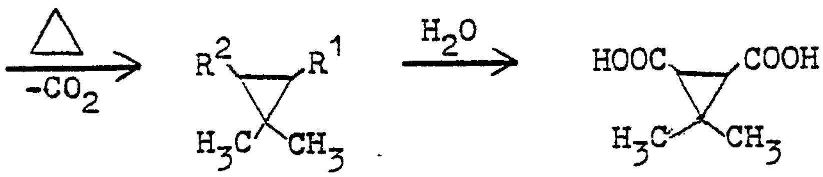


(VI)

Hierzu werden zunächst 3,3-Dimethyl-cyclopropan-1,1,2-tricarbonsäure-derivate (I) mit Basen, wie z.B. Natrium- oder Kalium-hydroxid, gegebenenfalls unter Verwendung von Verdünnungsmitteln, wie z.B. Methanol und Wasser, bei Temperaturen zwischen 0 und 80°C, vorzugsweise zwischen 10 und 50°C, umgesetzt. Zur Isolierung der hierbei gebildeten Verbindungen der Formel (V) wird angesäuert, das sich dabei abscheidende ölige Produkt in Methylenchlorid aufgenommen, die Lösung getrocknet, filtriert und eingeengt. Das zurückbleibende Rohprodukt kann ohne weitere Reinigung für die nächste Stufe eingesetzt werden.

Zur Decarboxylierung werden die Carbonsäuren der Formel (V), gegebenenfalls in Gegenwart von Verdünnungsmitteln, wie z.B. Dimethylformamid oder Dimethylsulfoxid, auf Temperaturen zwischen 120 und 200°C, erhitzt. Die Aufarbeitung und Isolierung der hierbei gebildeten Verbindungen der Formel (VI) wird nach üblichen Methoden durchgeführt:man gießt in Wasser und extrahiert mit einem mit Wasser nicht mischbaren organischen Lösungsmittel, wie z.B. Methylenchlorid, trocknet die Extrakte, engt ein und reinigt gegebenenfalls das Produkt durch Vakuumdestillation.

Partielle Hydrolyse und Decarboxylierung können auch kombiniert werden, indem man Verbindungen der Formel (I) mit angenähert äquimolaren Mengen Wasser in hochsiedenden organischen Lösungsmitteln, wie z.B. Dimethylformamid oder Dimethylsulfoxid, auf Temperaturen zwischen 100 und 250°C, vorzugsweise zwischen 120 und 200°C, erhitzt. Zur Aufarbeitung und Isolierung der hierbei gebildeten Produkte der Formel (VI) wird mit einem mit Wasser nicht mischbaren Lösungsmittel verdünnt (z.B. mit Toluol), mit Wasser gewaschen, getrocknet, eingeengt und gegebenenfalls unter vermindertem Druck destilliert.

Verbindungen der Formel (VI) können durch Hydrolyse, beispielsweise durch Erhitzen mit Alkalilaugen, wie z.B. 15%iger Natronlauge, auf Temperaturen zwischen 80 und 120°C, und anschließendes Ansäuern bei Raumtemperatur mit starken Säuren, wie z.B. Salzsäure, in 3,3-Dimethylcyclopropan-1,2-dicarbonsäure (Caronsäure) umgewandelt werden. Caronsäure fällt hierbei kristallin an und kann durch Filtration isoliert werden.

Caronsäure oder deren Ester können als Zwischenprodukte zur Herstellung von insektizid und akarizid wirksamen Pyrethroiden verwendet werden (vgl. Pestic. Sci. 7 (1976), 492-498; Tetrahedron Lett. 1978, 1847-1850).

Beispiel 1

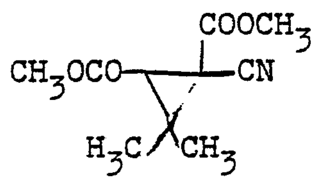

Zu einer Lösung von 19,9 g (0,2 Mol) Cyanessigsäuremethylester und 10,8 g (0,2 Mol) Natriummethylat in 50 ml Methanol werden bei 20°C 27,2 g (0,1 Mol) α,β-Dibrom-iso-valeriansäure-methylester getropft. Anschließend wird 5 Stunden auf 64°C erhitzt. Dann wird das Lösungsmittel im Vakuum abgezogen. Der Rückstand wird mit 100 ml Wasser und 100 ml Äther versetzt. Die Ätherphase wird abgetrennt, über Natriumsulfat getrocknet und eingedampft. Der Rückstand wird zweimal fraktioniert. Man erhält 12 g (57 % d. Theorie) 3,3-Dimethyl-1-cyano-cyclopropan-1,2-dicarbonsäure-dimethylester in Form eines farblosen Öls vom Siedepunkt 152° / 10 mm Hg.

Beispiel 2

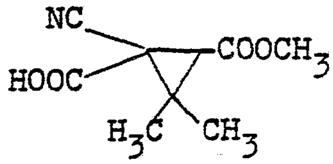

Eine Lösung von 21,1 g (0,1 Mol) 3,3-Dimethyl-1-cyano-cyclopropan-1,2-dicarbonsäure-dimethylester und 5,6 g Kaliumhydroxid in einem Gemisch von 50 ml Methanol und 25 ml Wasser wird 2 Stunden bei Raumtemperatur gerührt. Dann wird filtriert und das Filtrat mit verdünnter Salzsäure angesäuert. Es scheidet sich ein Öl ab, das in Methylenchlorid aufgenommen wird. Die Methylenchloridphase wird über Natriumsulfat getrocknet und dann eingeengt.
Man erhält 13 g (65 % d. Theorie) 3,3-Dimethyl-2-methoxycarbonyl-1-cyano-cyclopropan-1-carbonsäure in Form einer teilkristallinen Masse, die ohne Reinigung direkt weiterverarbeitet wird.

Beispiel 3

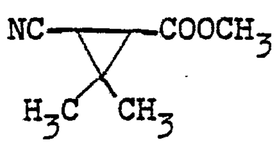

19,7 g (0,1 Mol) des nach Beispiel 2 erhaltenen Produktes werden in 80 ml Dimethylformamid 4 Stunden auf 140-150°C erhitzt.

Dann wird abgekühlt und das Reaktionsgemisch wird in 200 ml Wasser gegossen. Anschließend wird zweimal mit je 50 ml Methylenchlorid extrahiert.
Die vereinigten Methylenchloridextrakte werden über Natriumsulfat getrocknet und dann eingeengt.
Nach Fraktionierung erhält man 16 g (75 % d. Theorie) 3,3-Dimethyl-2-cyano-cyclopropan-1-carbonsäure-methylester in Form eines farblosen Öls vom Kp. 90-104$^{o}$ / 2 mm Hg.

Beispiel 4

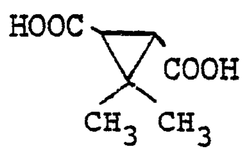

Eine Mischung von 50 ml 15 proz. Natronlauge und 8,5 g (0,05 Mol) 3,3-Dimethyl-2-cyano-cyclopropan-1-carbensäure-methylester wird 18 Stunden unter Rückfluß gekocht. Dann kühlt man das Gemisch auf 10$^{o}$C ab, gibt konz. Salzsäure zu bis ein pH-Wert von ca. 2 erreicht ist und kühlt dann 1 Stunde im Eisbad. Das ausgefallene Produkt wird abgesaugt und mit Eiswasser nachgewaschen. Man erhält so 7,3 g (92 % d. Theorie) trans-3,3-Dimethylcyclopropan-1,2-dicarbonsäure in Form eines farblosen Pulvers mit dem Schmelzpunkt 217$^{o}$C.

Beispiel 5

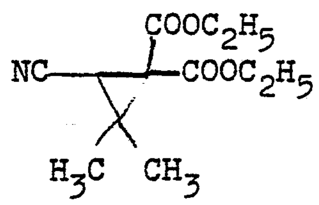

Eine Lösung von 24,1 g (0,1 Mol) α,ß-Dibrom-isovalerinsäurenitril in 20 ml Dimethylformamid wird zu einer Mischung aus 35 g (0,1 Mol) Malonsäurediäthylester, 30 g Kaliumcarbonat und 120 ml Dimethylformamid tropfenweise gegeben. Die Innentemperatur steigt bei der hierbei ablaufenden exothermen Reaktion von zunächst etwa 25°C auf etwa 40°C an. Das Reaktionsgemisch wird 15 Stunden bei 20°C gerührt, in 200 ml Toluol aufgenommen, zweimal mit je 200 ml Wasser gewaschen, getrocknet und destilliert. Bei einer ersten Destillation erhält man 13,1 g (55 % d. Theorie) 3,3-Dimethyl-2-cyano-cyclopropan-1,1-dicarbonsäure-diäthylester als nach gaschromatographischer Analyse 75%iges Produkt mit Siedetemperatur 100-140°C / 2 mBar.
Eine zweite Destillation ergibt 9,7 g (41 % d. Theorie) reines Produkt.

Beispiel 6

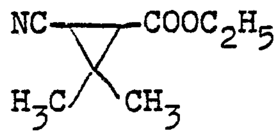

Eine Mischung aus 71,7 g (0,3 Mol) 3,3-Dimethyl-2-cyano-cyclopropan-1,1-dicarbonsäure-diäthylester, 6 ml Wasser und 100 ml Dimethylformamid wird 20 Stunden unter Rückfluß zum Sieden erhitzt. Zur Aufarbeitung wird mit Toluol verdünnt, zweimal mit je 300 ml Wasser gewaschen, getrocknet und destilliert. Man erhält 30,6 g (61 % der Theorie) 3,3-Dimethyl-2-cyano-cyclopropan-1-carbonsäure-äthylester vom Siedepunkt 87-95°C / 2 mBar.

Beispiel 7

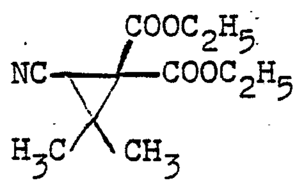

19,2 g α-Brom-ß,ß-dimethyl-acrylsäurenitril werden zu einer Mischung aus 20 g Malonsäure-diäthylester, 35 g Kaliumcarbonat und 80 ml Dimethylformamid tropfenweise gegeben. Die Innentemperatur steigt bei der hierbei ablaufenden exothernen Reaktion von 25 auf 30°C an. Das Reaktionsgemisch wird 15 Stunden bei 20°C gerührt, in Methylenchlorid aufgenommen, mit Wasser gewaschen, getrocknet und mehrfach destilliert.
Man erhält 4,1 g (14 % der Theorie) 3,3-Dimethyl-2-Cyano-1,1-dicarbonsäure-diäthylester mit der Siedetemperatur 115-125°C / 2 mBar.

Beispiel 8

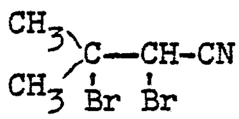

32 g (0,2 Mol) Brom werden zu einer Lösung von 16,2 g (0,2 Mol) ß,ß-Dimethyl-acrylsäurenitril in 100 ml Tetrachlorkohlenstoff tropfenweise gegeben. Bei der hierbei ablaufenden exothernen Reaktion steigt die Innentemperatur von 25 auf 35°C. Nach zweistündigem Rühren wird destilliert. Man erhält 41 g (85 % der Theorie) α,ß-Dibrom-iso-valeriansäurenitril vom Siedepunkt 70°C / 2 mBar.

Beispiel 9

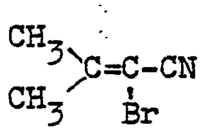

Eine Lösung von 101 g (0,42 Mol) α,ß-Dibrom-iso-valeriansäurenitril in 80 ml Dimethylformamid wird zu einer Mischung 63 g Kaliumcarbonat und 210 ml Dimethylformamid tropfenweise gegeben. Bei der hierbei ablaufenden exothernen Reaktion steigt die Innentemperatur von 20 auf 35°C an. Das Reaktionsgemisch wird eine Stunde bei Raumtemperatur gerührt, mit 300 ml Äther verdünnt und zweimal mit je 300 ml Wasser gewaschen. Nach Trocknen der organischen Phase mit Natriumsulfat und fraktionierter Destillation erhält man 49,8 g (74 % der Theorie) α-Brom-ß,ß-dimethyl-acrylsäurenitril vom Siedepunkt 57°C / 15 mBar.

Patentansprüche

1. Verfahren zur Herstellung von 3,3-Dimethyl-cyclopropan-1,1,2-tricarbonsäure-derivaten der Formel (I)

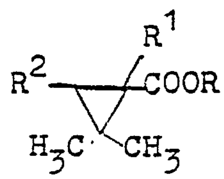

(I)

in welcher

R für Alkyl steht und

$R^1$ und $R^2$ für Alkoxycarbonyl oder Cyano stehen mit der Maßgabe, daß wenigstens einer dieser Reste für Cyano steht,

dadurch gekennzeichnet, daß man α-Halogencarbonsäurederivate der Formel II

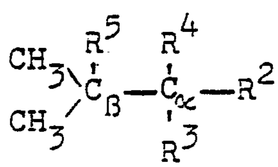

(II)

in welcher

$R^2$ für Alkoxycarbonyl oder Cyano steht,

$R^3$ für Brom oder Chlor steht,

$R^4$ für Wasserstoff steht oder zusammen mit $R^5$ für eine zusätzliche Bindung zwischen $C_\alpha$ und $C_\beta$ steht, und

$R^5$ für Brom oder Chlor steht oder zusammen mit $R^4$ für eine zusätzliche Bindung zwischen $C_\alpha$ und $C_\beta$ steht,

mit Malonsäurederivaten der Formel III

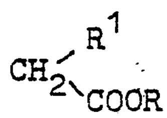 (III)

in welcher
R und $R^1$ die oben angegebenen Bedeutungen haben,

gegebenenfalls in Gegenwart einer Base und gegebenenfalls unter Verwendung eines Verdünnungsmittels bei Temperaturen zwischen 0 und 100°C umsetzt;

2. α-Halogen-nitrile der Formel IIa

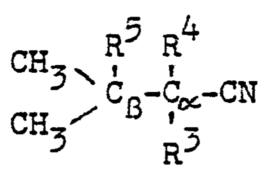 (IIa)

in welcher

$R^3$ für Brom oder Chlor steht,

$R^4$ für Wasserstoff steht oder zusammen mit $R^5$ für eine zusätzliche Bindung zwischen $C_\alpha$ und $C_\beta$ steht, und

$R^5$ für Brom oder Chlor steht, oder zusammen mit $R^4$ für eine zusätzliche Bindung zwischen $C_\alpha$ und $C_\beta$ steht;

3. Verfahren zur Herstellung von $\alpha$-Halogen-nitrilen der Formel IIa (oben), dadurch gekennzeichnet, daß man ß,ß-Dimethyl-acrylsäurenitril der Formel IV

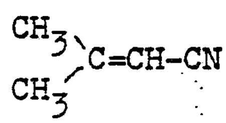

(IV)

mit Brom oder Chlor, gegebenenfalls in Gegenwart eines Verdünnungsmittels, bei Temperaturen zwischen -20 und +50°C umsetzt und gegebenenfalls die Halogenierungsprodukte der Formel IIa ($R^4$=H, $R^5$=Cl,Br) zur Eliminierung von Halogenwasserstoff bei Temperaturen zwischen 0 und 100°C mit Basen behandelt.

0021115

Nummer der Anmeldung

Europäisches Patentamt

# EUROPÄISCHER RECHERCHENBERICHT

EP 80103023.0

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - C - 965 580 (BAYER)<br>+ Patentanspruch +<br>-- | 1,3 | C 07 C 121/46<br>C 07 C 121/16<br>C 07 C 121/30 |
| | DE - A1 - 2 724 734 (AMERICAN CYANAMID)<br>+ Patentanspruch 1 +<br>-- | 1 | |
| | DE - A1 - 2 926 852 (ICI)<br>+ Patentanspruch 1; Seite 10, letzter Satz; Seite 5 +<br>-- | 1 | RECHERCHIERTE SACHGEBIETE (Int. Cl. 3) |
| | DE - A1 - 2 621 830 (ICI)<br>+ Patentansprüche 1,5 +<br>-- | 1,2 | C 07 C 121/00<br>C 07 C 69/00 |
| | DE - A1 - 2 730 755 (ICI)<br>+ Patentanspruch 1 +<br>---- | 1-3 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| WIEN | 28-08-1980 | REIF |